(19) Europäisches Patentamt
European Patent Office
Office européen des brevets

(11) Numéro de publication: **0 424 243 B1**

(12) # FASCICULE DE BREVET EUROPEEN

(45) Date de publication du fascicule du brevet: **23.08.95**

(51) Int. Cl.[6]: **C07D 263/24**, A61K 31/42, C07D 413/10

(21) Numéro de dépôt: **90402891.7**

(22) Date de dépôt: **16.10.90**

Le dossier contient des informations techniques présentées postérieurement au dépôt de la demande et ne figurant pas dans le présent fascicule.

(54) **Dérivés d'aryl-3 oxazolidinone, leur procédé de préparation et leur application en thérapeutique.**

(30) Priorité: **17.10.89 FR 8913555**

(43) Date de publication de la demande:
**24.04.91 Bulletin 91/17**

(45) Mention de la délivrance du brevet:
**23.08.95 Bulletin 95/34**

(84) Etats contractants désignés:
**AT BE CH DE DK ES FR GB GR IT LI LU NL SE**

(56) Documents cités:
**FR-A- 2 458 547**
**GB-A- 2 003 151**
**GB-A- 2 076 813**

(73) Titulaire: **DELALANDE S.A.**
**32, rue Henri Regnault**
**F-92400 Courbevoie (FR)**

(72) Inventeur: **Jarreau, François-Xavier**
**5, rue Louis Hervé**
**F-78000 Versailles (FR)**
Inventeur: **Rovei, Vincenzo**
**44, rue Danton**
**F-92500 Rueil Malmaison (FR)**
Inventeur: **Koenig, Jean-Jacques**
**99, avenue du Général de Gaulle**
**F-78600 Maisons Laffitte (FR)**
Inventeur: **Schoofs, Alain**
**242, rue de la Croix-Nivert**
**F-75015 Paris (FR)**

(74) Mandataire: **Kedinger, Jean-Paul et al**
**c/o Cabinet Malemont**
**42, avenue du Président Wilson**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

GB-A-2 076 813 décrit des N-aryl-oxazolidinones-2 présentant une activité antidépressive.

La présente invention a pour objet de nouveaux dérivés acétal d'aryl-3 oxazolidinone-2, leur procédé de préparation et leur application en thérapeutique.

Ces dérivés répondent plus précisément à la formule :

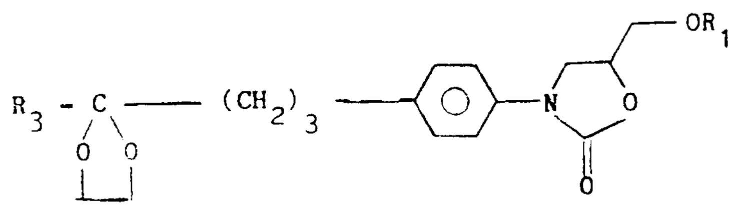

(I)

où :

- $R_1$ représente H ou alkyle en $C_1$-$C_4$ ;
- $R_3$ représente un groupe alkyle en $C_1$-$C_4$ ou $CF_3$ ;

Par ailleurs, il convient de souligner que les dérivés (I) comportent un atome de carbone asymétrique. Ils peuvent donc se présenter sous la forme d'énantiomères ou sous la forme d'un mélange d'énantiomères, y compris les formes racémiques. La présente invention s'étend par conséquent aux diverses formes ainsi définies.

L'invention concerne également les sels d'addition d'acide des dérivés (I) qui comportent un groupe salifiable. Ces sels peuvent être formés avec des acides minéraux tels que l'acide chlorhydrique, sulfurique, phosphorique ou avec des acides organiques tels que l'acide fumarique, maléique, succinique, oxalique, citrique ou tartrique.

La formule (I) ci-dessus couvre notamment les dérivés pour lesquels $R_1$ = H ou $CH_3$ ; et $R_3$ = alkyle en $C_1$-$C_4$.

On citera en particulier :

- les dérivés pour lesquels $R_1$ = $CH_3$ ; et $R_3$ = $CH_3$ ou $CF_3$ ;

La présente invention a par ailleurs pour objet les procédés de préparation des dérivés (I) et de leurs sels d'addition d'acide.

Ces procédés sont essentiellement basés sur deux voies générales de synthèse.

La première de ces voies comprend l'élaboration d'une entité comportant le fragment oxazolidinone-2 (schéma 1), suivie du greffage sur cette entité de la chaîne comportant le reste acétal (schéma 2).

La deuxième de ces voies comprend, à l'inverse, d'abord l'élaboration de la chaîne comportant le reste acétal (schéma 3), suivie de l'élaboration et greffage sur ce reste acétal d'une entité comportant le fragment oxazolidinone-2 (schémas 4 et 5).

Ces cinq schémas sont représentés ci-après. Sauf indication contraire, les symboles $R_1$ et $R_3$ apparaissant dans ces schémas, ont la même signification que dans la formule (I).

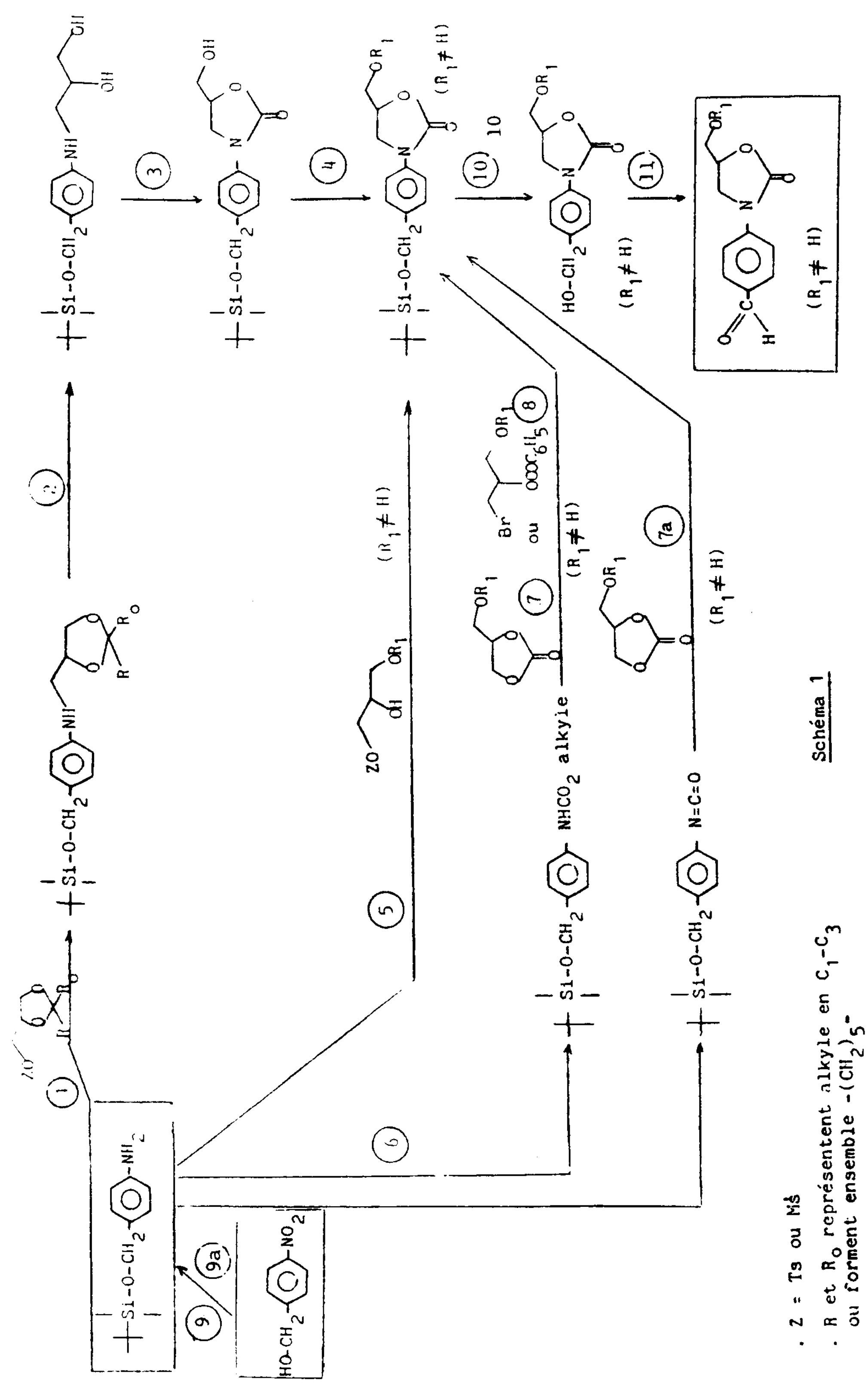

Schéma 1

. Z = Ts ou Ms

. R et $R_o$ représentent alkyle en $C_1$-$C_3$ ou forment ensemble $-(CH_2)_5-$

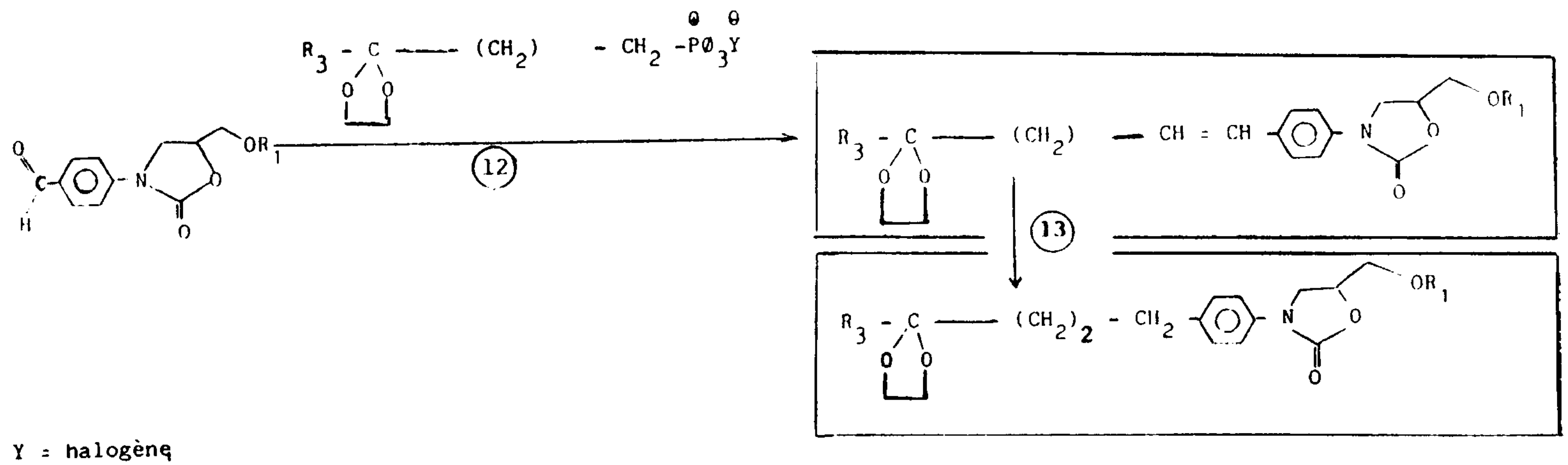

Y : halogène
$R_1 \neq H$

**Schéma 2**

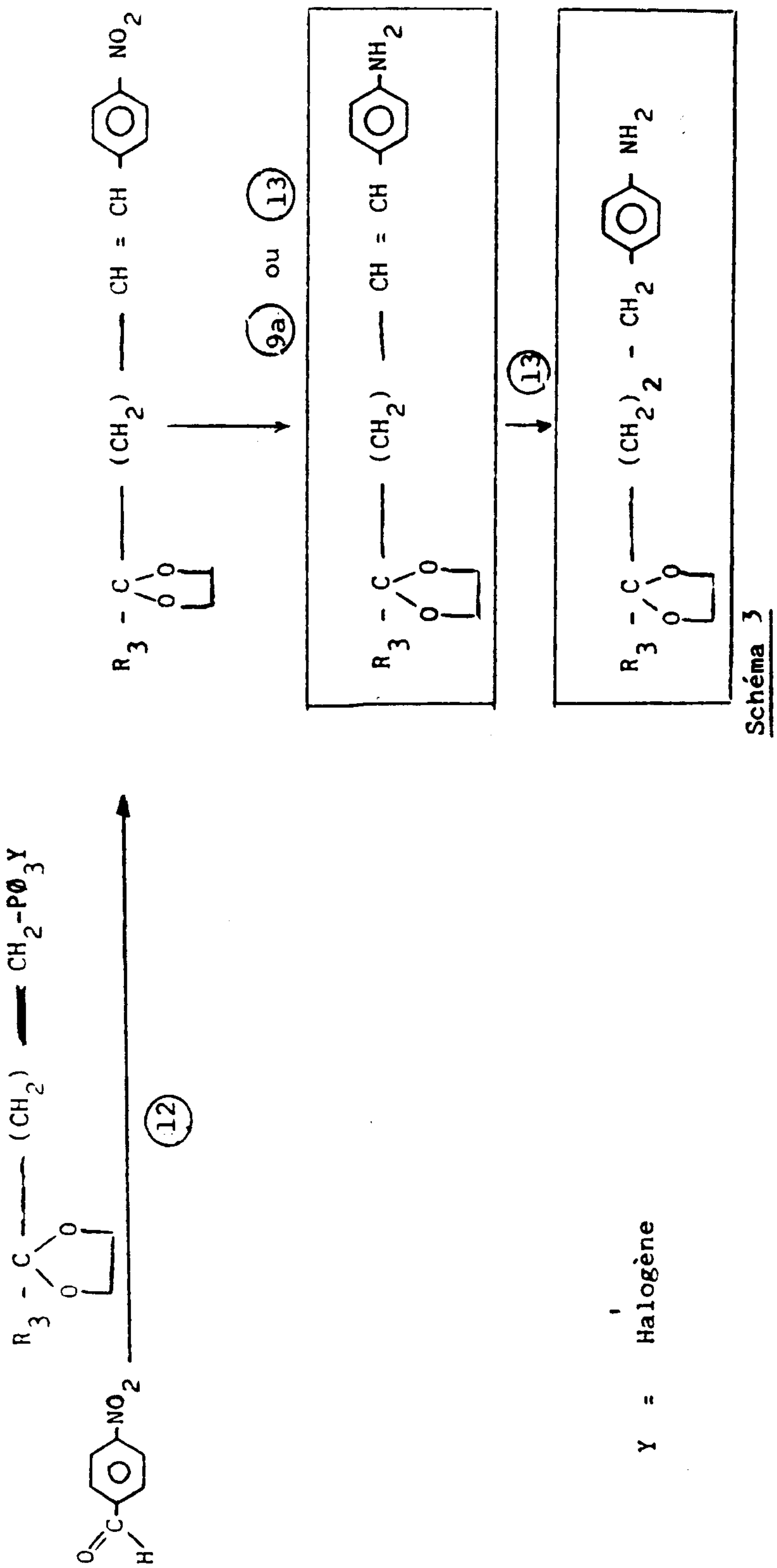


Y = Halogène

Schéma 3

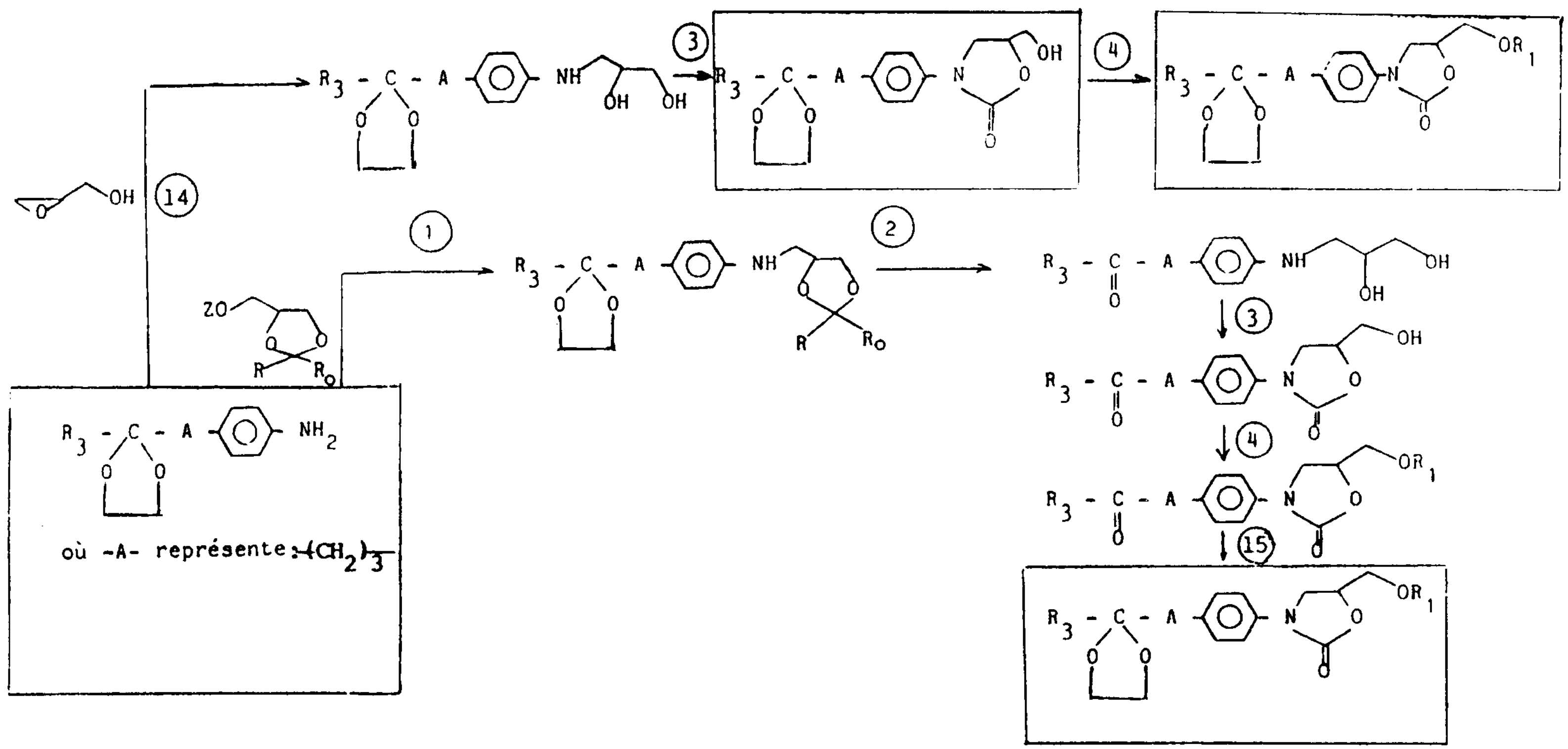


. Z = Ts ou Ms

. $R_1 \neq H$

. dans la succession d'étapes (1), (2), (3), (4) et (15), $R_3 \neq CF_3$

. R et $R_o$ représentent alkyle en $C_1$-$C_4$ ou forment ensemble $-(CH_2)_5-$

**Schéma 4**

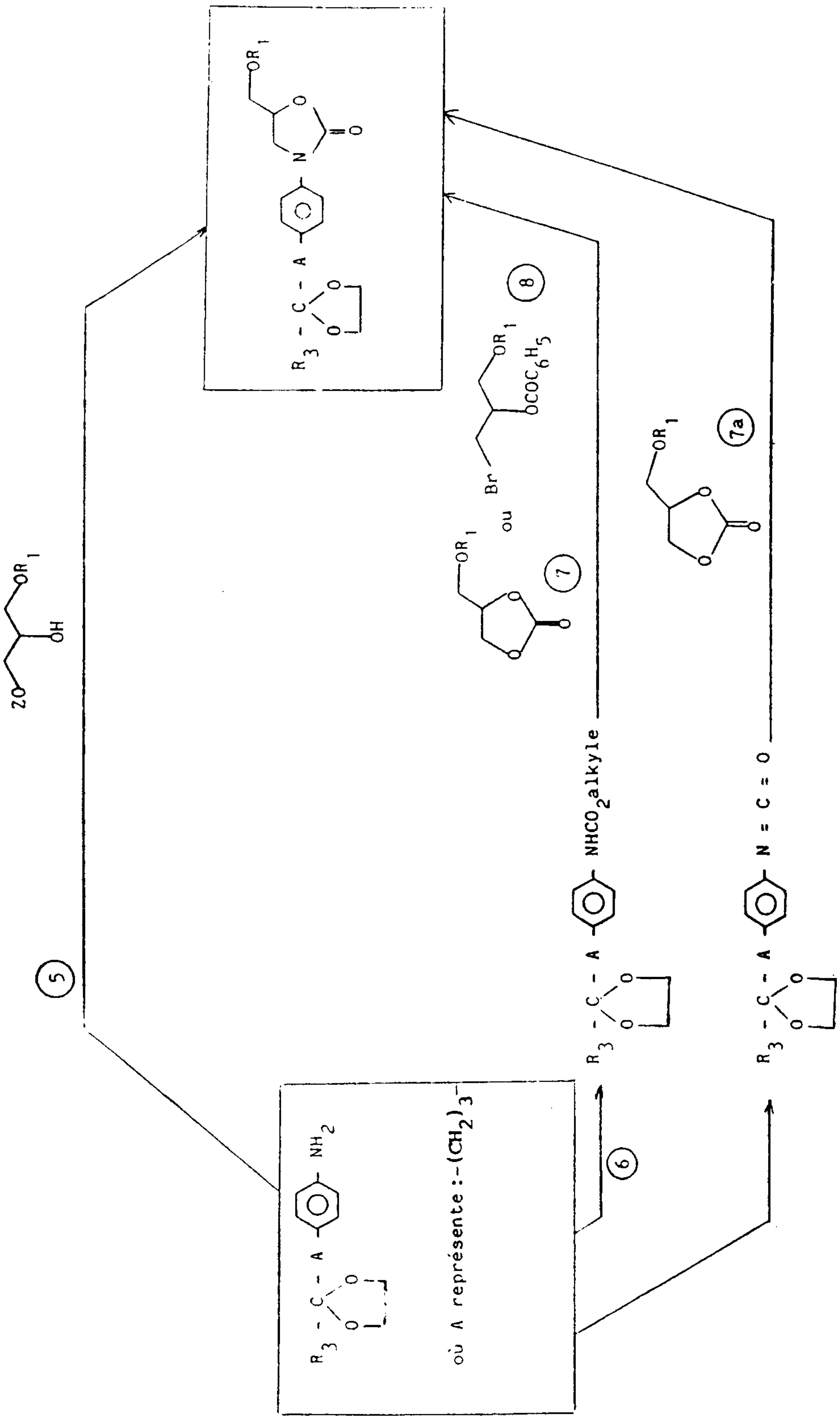


Schéma 5

$R_1 \neq H$

Z = Ts ou Ms

Par ailleurs, les composés de formules :

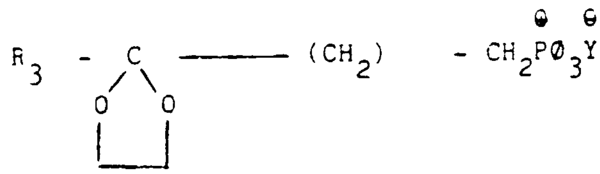 (schéma 2)

sont obtenus conformément au schéma 6 ci-après.

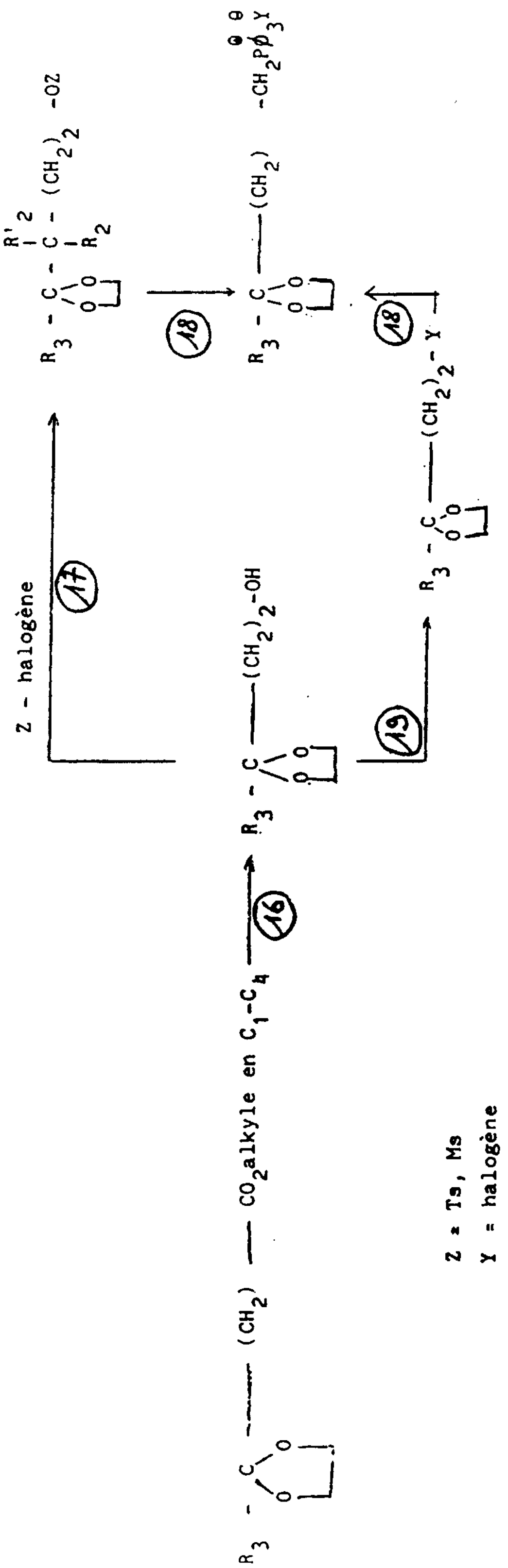


Schéma 6

Les indices ① à ⑲ apparaissant dans les schémas ci-dessus, ont les significations suivantes :

① Condensation soit dans un solvant aprotique anhydre comme le toluène, par chauffage, avec ou sans catalyseur comme le bromure d'hexadécyl tributyl phosphonium ou un halogénure d'ammonium quaternaire tel que le bromure de benzyl triéthylammonium, soit sans solvant en présence de triéthylamine entre 130-150° C.

② Hydrolyse par un acide aqueux notamment l'acide chlorhydrique 6N en présence d'un solvant organique comme la méthyléthylcétone.

③ Condensation avec un carbonate d'alkyle en $C_1$-$C_4$ notamment le carbonate de diéthyle dans un solvant anhydre comme le toluène en présence d'alcoolate de métal alcalin comme le méthylate de sodium.

④ Alcoylation par un halogénure (bromure ou chlorure) d'alkyle en $C_1$-$C_4$ dans les conditions de transfert de phase notamment soude-chlorure de méthylène ou toluène, en présence d'un ammonium quaternaire comme le bromure ou l'hydrogénosulfate de tétrabutylammonium.

⑤ Condensation en présence de phosgène et d'une base notamment la diméthylaniline dans un solvant organique comme le chlorure de méthylène ou le dichloroéthane ; puis cyclisation par chauffage dans un solvant organique notamment alcoolique comme l'éthanol en présence d'une base notamment la potasse.

⑥ Condensation avec un chloroformiate d'alkyle, comme le chloroformiate d'éthyle, en présence d'une base notamment $NaHCO_3$ dans un mélange solvant eau-THF, à température ambiante.

⑦ Condensation à chaud (vers 150° C) en présence d'une base comme $K_2CO_3$. La réaction conserve la stéréochimie.

(7a) Condensation dans le toluène en présence de LiBr et de $nBu_3PO$.

⑧ Condensation en présence d'une base notamment NaH dans un solvant aprotique comme le THF à 55° C-60° C.

⑨ O.silylation de l'alcool dans un solvant organique aprotique comme le THF en présence d'une base notamment l'imidazole, et de terbutylediméthylchlorosilane.

(9a) Réduction du dérivé nitré par la poudre de fer en présence de chlorure d'ammonium.

⑩ Hydrolyse dans un solvant organique notamment le THF en présence de fluorure, notamment de tétrabutylammonium.

⑪ Oxydation en présence de chlorure d'oxalyle, de DMSO et d'une base, notamment la triéthylamine, dans un solvant organique aprotique comme le chlorure de méthylène.

⑫ Condensation en présence d'une base notamment $K_2CO_3$ et de formamide dans un solvant organique notamment le dioxanne, de préférence au reflux,

ou

Condensation en présence de LDA (lithium diisopropylamide) dans un mélange de solvants notamment DMSO/THF.

⑬ Hydrogénation sous pression atmosphérique d'hydrogène dans un solvant organique notamment l'acétate d'éthyle en présence d'un catalyseur comme le palladium sur carbone à 10 % humidifié ou non,

ou

Hydrogénation sous pression d'hydrogène notamment sous 5 atmosphères, en présence de palladium sur carbone à 10 % humidifié ou non dans un solvant alcoolique, notamment l'éthanol,

ou

Hydrogénation sous pression d'hydrogène notamment sous 9 atmosphères, en présence de palladium sur carbone à 10 % humidifié ou non dans un solvant alcoolique, notamment l'éthanol.

⑭ Condensation dans un solvant alcoolique comme l'éthanol ou l'isopropanol à température ambiante ou par chauffage au reflux, en absence ou en présence de catalyseur comme le chlorure de cobalt.

⑮ Acétalisation en présence de silice greffée aminopropyle sous forme de chlorhydrate, par $HO-(CH_2)_2-OH$, dans un solvant aprotique notamment le chlorure de méthylène et avec ou sans orthoformiate d'éthyle,

ou

Acétalisation par $HO-(CH_2)_2-OH$, au reflux dans un solvant organique aprotique notamment le toluène, en présence de l'acide paratoluène sulfonique, en éliminant l'eau formée.

⑯ Réduction dans un solvant organique aprotique comme le diméthoxyéthane en présence de borohydrure de lithium ou dans un solvant organique comme le THF en présence de $LiAlH_4$.

⑰ Condensation dans un solvant organique notamment la pyridine ou $CH_2Cl_2$ en présence d'une base notamment la diméthylamino-4 pyridine ou $Et_3N$.

⑱ Conformément à Helv. Chim. Acta 59, 755 (1976).

⑲ Conformément à Can. J. Chem. 1968, 46, 86.

Les préparations suivantes sont données à titre d'exemple pour illustrer l'invention.

Exemple 1 :
[(oxo-4 pentyl)-4 phényl]-3 hydroxyméthyl-5 (R) oxazolidinone (numéro de code 230116)

Stade 1 :
(p-nitrocinnamyl)-2 méthyl-2 dioxolane-1,3 (numéro de code 230111).

A 62,8 mmole de LDA dans 226,4 ml de THF on ajoute à 0° C une solution de 28,8 g (62,2 mmole) de bromure de (méthyl-2 dioxolane-yl-2 éthyl-2) triphénylphosphonium dans 60 ml de DMSO, goutte à goutte. Après 1 h à 0°C, on ajoute 7,8 g (51,6 mmoles) de p-nitro benzaldéhyde en solution dans 40 ml de THF. Le milieu réactionnel est hydrolysé par une solution saturée de $NH_4Cl$ et est extrait à l'éther éthylique. La phase organique est séchée sur $Na_2SO_4$ et concentrée. Après purification par chromatographie flash (silice, éluant : heptane : 70 ; acétate d'éthyle : 30) le produit est obtenu avec un rendement de 48 %. RMN$^1$H ($CDCl_3$)δppm : 1,3 (3H) ; 2,6 (2H) ; 4 (4H) ; 5,7-6,5 (2H) ; 7,4 (2H) : 8,1 (2H).

Stade 2 :
(amino-4 cinnamyl)-2 méthyl-2 dioxolane-1,3 (numéro de code 230112).

A une solution de 8 g (32 mmoles) du composé 230111 dans 100 ml d'éthanol en présence de 0,8 g de Pd/C à 10 %, dans un autoclave, on fait passer un courant d'hydrogène sous 5 atmosphères pendant 4 h. Après filtration, concentration, purification par chromatographie flash (silice, éluant : heptane : 50 ; acétate d'éthyle : 50) le produit est obtenu avec un rendement de 89 %. F : < 50°C ;
RMN$^1$H ($CDCl_3$)δppm : 1,35 (3H) ; 2,4-2,8 (2H) ; 3,6 (2H éch.) ; 4 (4H) ; 5,5-6,3 (2H) ; 6,6 (2H) ; 7,2 (2H) ; IR (microcellule) $\nu cm^{-1}$ : 3460, 3440.

Stade 3 :
[(amino-4 phényl)-3 propyl]-2 méthyl-2 dioxolane-1,3 (numéro de code 230113).

Une solution de 15,4 g (70,23 mmoles) du composé 230112 dans 100 ml d'éthanol en présence de 1,5 g de Pd/C à 10 % est placée dans un autoclave ; on fait arriver un courant d'hydrogène sous 9 atmosphères pendant 1 h à 50° C. Après filtration et concentration, on obtient 15,5 g de produit attendu (liquide).
RMN$^1$H ($CDCl_3$)δppm : 1,25 (3H) ; 1,6 (4H) ; 2,45 (2H) ; 3,5 (2H, éch.) ; 3,85 (4H) ; 6,55 (2H) ; 6,9 (2H). IR (microcellule , $\nu cm^{-1}$) : 3460, 3350.

Stade 4 :
[[Méthyl-2 dioxolane-1,3 yl-2)-3 propyl]-4 phényl]dioxa-1,4 spiro [4,5] décane méthanamine-2 (R) (numéro de code 230114)

A un mélange d'1 g (4,5 mmoles) du composé 230113 et 1,62 g (4,97 mmoles) de tosylate du dioxa-1,4 spiro [4,5] décane méthanol-2 (S), on ajoute 0,73 g (1 ml, 7,23 mmoles) de triéthylamine et on chauffe à 140°C pendant 5 h. Le milieu réactionnel est repris dans l'eau et extrait à l'acétate d'éthyle. La phase organique est lavée à l'eau salée, puis, séchée sur $Na_2SO_4$. On obtient le produit sous forme liquide avec un rendement de 59 % après chromatographie flash (silice, éluant : heptane : 40 : acétate d'éthyle : 60).
RMN$^1$H ($CDCl_3$)δppm : 1,2 (3H) ; 1,5 (10H) ; 1,6 (4H) ; 2,4 (3H) ; 3,15 (3H) ; 3,8 (4H) ; 3,6-4,5 (3H). IR (microcellule) $\nu cm^{-1}$ : 3400 ; $[\alpha]_D^{20}$ = - 2,9° (C = 1, MeOH).

Stade 5 :
[(oxo-4 pentyl)-4 phényl]-amino propanediol-1,2 (R) (numéro de code 230115)

A une solution de 1,7 $10^{-3}$ mole du composé 230114 dans 3,5 ml de THF, on ajoute, goutte à goutte, 3,5 ml d'acide chlorhydrique 6N. Après 1 h, le milieu réactionnel est versé sur l'eau et extrait à l'acétate d'éthyle. La phase organique est lavée à l'eau, séchée sur $Na_2SO_4$ et concentrée.
RMN$^1$H ($CDCl_3$)δppm : 1,8 (2H) ; 2 (3H) ; 2,4 (4H) ; 2,7-3,3 (3H) ; 3,1 (3H) ; 3,6 (2H) ; 3,3 (1H) ; 6,5 (2H) ; 6,9 (2H).

Stade 6 :
[(oxo-4 pentyl)-4 phényl]-3 hydroxyméthyl-5 (R) oxazolidinone-2 (numéro de code 230116).

On chauffe au reflux une solution de 0,0139 mole de 230115 dans 50 ml de toluène et à 90° C on ajoute 0,0159 mole de carbonate de diéthyle, puis petit à petit 0,32 ml d'une solution de méthylate de sodium à 4,3 moles/litre.

Après avoir distillé l'alcool, le milieu réactionnel est concentré, le résidu est repris dans l'acétate d'éthyle. La phase organique est lavée à l'eau, sechée et concentrée. Le produit est obtenu après chromatographie sur colonne (silice, éluant : $CH_2Cl_2$)
F = 110°C ; $[\alpha]_D^{20}$ = - 50,7° (C = 1, MeOH) ; RMN$^1$H ($CDCl_3$)δppm : 1,8 (2H) ; 2,05 (3H) ; 2,2-2,7 (4H) ; 2,75 (1H) ; 3,65-4,10 (2H) ; 4,65 (1H) ; 7,1 (2H) ; 7,4 (2H) ; IR (KBr) $\nu cm^{-1}$ : 3460, 1720.

Exemple 2 :
[ (oxo-4 pentyl )-4 phényl ]-3 méthoxyméthyl-5 (R) oxazolidinone-2(numéro de code 230083).

A une suspension de 9,44 mmoles de 230116 dans 40 ml de toluène et 40 ml de NaOH à 50 %, on ajoute 0,3 g de bromure de tétrabutylammonium et 2,7 ml (28,3 mmoles) de sulfate de méthyle. Après 30 mn, le milieu réactionnel est versé sur l'eau et extrait à l'acétate d'éthyle. La phase organique est lavée à l'eau saturée de NaCl, séchée et concentrée. Après chromatographie (silice, éluant : acétate d'éthyle 50 - heptane 50), on obtient le produit. Rendement : 100 %.
F : < 50°C ; $[\alpha]_D^{20}$ = - 56,9° (C = 1, MeOH) ; RMN$^1$H ($CDCl_3$)δppm : 1,9(2H); 2,1 (3H) ; 2,45 (4H) ; 3,4 (3H) ; 3,6 (2H) ; 3,9 (2H) ; 4,7 (1H) ; 7,1 (2H) ; 7,4 (2H) ; IR (KBr) $\nu cm^{-1}$ : 1750, 1710.

Exemple 3 :
[[(méthyl-2 dioxolane-1,3 yl-2)-3 propyl]-4 phényl]-3 méthoxyméthyl(R)-5 oxazolidinone-2 (numéro de code 230084).

Méthode 1

A une solution de 1,83 g (6,28 mmoles) du composé 230083 (exemple 2)dans 25 ml de toluène on ajoute 0,382 g (6,28 mmoles) d'éthylène glycol et on chauffe au reflux 12 h en présence d'acide p-toluène sulfonique et en éliminant l'eau. Le milieu réactionnel est concentré. Le résidu est repris dans $CH_2Cl_2$, la phase organique est lavée au $NaHCO_3$ puis à l'eau, séchée et concentrée. Le produit est purifié par HPLC (silice, éluant : éther isopropylique : 65 ; heptane : 25 ; méthanol : 10).
F = 81°C ; $[\alpha]_D^{20}$ = - 49° (C = 1, MeOH) ; IR (KBr) $\nu cm^{-1}$: 1740 ; RMN$^1$H ($CDCl_3$)δppm : 1,25 (3H) ; 1,65 (4H) ; 2,55 (2H) ; 3,4 (3H) ; 3,6 (2H) ; 3,9 (6H) ; 4,65 (1H) ; 7,1 (2H) ; 7,4 (2H).

Méthode 2

Stade 1
Diméthyl-2,2 méthoxyméthyl-4 (S) dioxolane
(numéro de code 370486)

A 910 ml d'eau, on ajoute 910 g de NaOH en pastille, puis, à température ambiante, 5 l de $CH_2Cl_2$, 44,4 g (0,195 mole) de chlorure de benzyl triéthylammonium, 8.558,6 g (6,5 moles) de diméthyl-2,2 hydroxyméthyl-3 (S) dioxolane et 1.229,5 g (9,75 moles) de sulfate de diméthyle. Le milieu réactionnel est agité 12 h et versé sur l'eau. La phase organique est concentrée Le produit est distillé $Eb_{10}$ = 45° C $[\alpha]_D^{20}$ = + 7,9° (C = 4, $CH_3OH$) ; IR (microcellule) $\nu cm^{-1}$ = 2996, 2940, 2820, 1380, 1370, 840 ; RMN$^1$H ($CDCl_3$)δppm = 1,8 (3H) ; 1,4 (3H) ; 3,35 (3H) ; 3,4-4,4 (3H) ; 4 (2H). [J.A.C.S., 79, 1990 (1957)].

Stade 2
Méthoxy-3 propane diol-1,2 (R) (numéro de code 370487)

On chauffe une solution de 950,3 g (6,5 moles) de 370486 dans 450 ml d'eau à 60° C et on ajoute 3,2 ml d'acide chlorhydrique concentré, puis 9 ml de triéthylamine et le milieu réactionnel est concentré et distillé avec un rendement de 84 %. $Eb_1$ = 66° C ; $[\alpha]_D^{20}$ =-6,4° (C = 4, $CH_3OH$) ; IR (microcellule) $\nu cm^{-1}$ : 3500-3300, 2960, 2945, 2910 ; RMN$^1$H ($DMSOd_6$)δppm : 3,2-3,7 (8H) ; 4,5 (2H éch). [J.A.C.S., 79, 1990 (1957].

Stade 3
Tosylate de méthoxy-3 propanediol-1 ,2 (S)
(numéro de code 370488)

Une solution de 371,4 g (3,5 moles) de 370487 dans 100 ml de toluène est refroidie à 13° C et on ajoute 565 ml de pyridine, puis, petit à petit, une solution de 700,6 g (3,675 moles) de chlorure de paratoluène sulfonique dans 775 ml de toluène. Le milieu réactionnel est alors agité pendant 12 h et versé sur l'eau. La phase organique est lavée à l'acide chlorhydrique 2N et concentrée. Le produit est obtenu avec un rendement de 58 % après chromatographie (silice, éluant : $CH_2Cl_2$ : 50 éther de pétrole : 50). $[\alpha]_D^{20}$ = + 5,3° (C = 4, $CH_3OH$) ; IR (microcellule) $\nu cm^{-1}$ : 3500, 1335, 1185, 1170, ; RMN$^1$H ($CDCl_3$)$\delta$ppm : 2,4 (3H) ; 3,1 (1H éch.) ; 3,2-3,6 (5H) ; 3,8-4,2 (3H).

Stade 4
[[(méthyl-2 dioxolane-1,3 yl-2)-3 propyl]-4 phényl]-3 méthoxyméthyl-5 (R) oxazolidinone-2
(numéro de code 230084)

A 8,9 g (0,0887 mole) de phosgène dans 120 ml de dichloroéthane, on ajoute 15,4 g (0,059 mole) de 370488, puis 13,3 g((0.0887 mole) de diméthylaniline en solution dans 20 ml de dichloroéthane. Le milieu réactionnel est agité 1 h 30 à 50° C. Après refroidissement, celui-ci est lavé à l'eau glacée et séché sur sulfate de sodium. Cette solution est additionnée à une solution de 13 g (0,059 mole) de 230113 (exemple 1-stade 3) et 7,2 g (0,059 mole) de diméthyl amino-4 pyridine dans 200 ml de dichloroéthane. Le milieu réactionnel est alors chauffé au reflux 30 mn, refroidi et versé sur l'eau. La phase organique est lavée avec une solution de bicarbonate de sodium, séchée et concentrée. Le produit est obtenu après chromatographie (silice, éluant : éther isopropylique : 65, heptane : 25 ; $CH_3OH$ : 10) avec un rendement de 47 % et possède les mêmes caractères physiques que ceux obtenus par la méthode 1.

Méthode 3

Stade 1
Méthoxyméthyl-4 dioxolane-1,3 one-2 (S)
(numéro de code 360287)

On chauffe un mélange de 14 g (0,132 mole) de 370487 (méthode 2-stade 2), de 31,16 g (0,264 mole) de carbonate de diéthyle en présence de 0,108 g d'hydrure de sodium à 50 % jusqu'à distillation de l'alcool formé. Lorsque la réaction est complète ,le produit attendu est distillé $Eb_{0,3}$ : 117° C ; Rendement : 93 % ; $[\alpha]_D^{20}$ = : - 32,2° (C = 1, $CH_2Cl_2$) ; IR (microcellule) $\nu_{CO}$ : 1790 $cm^{-1}$ ; RMN$^1$H ($CDCl_3$)$\delta$ppm : 3,4 (3H) ; 3.6 (2H) ; 4.3-4.9 (3H).

Stade 2
[(éthoxycarbonyl-amino)-4 phénylpropyl]-2 méthyl-2 dioxolane-1,3
(numéro de code 360274)

A une solution de 6 g (0,027 mole) du composé 230113 (exemple 1-stade 3) dans un mélange de 63 ml de THF et 7 ml d'eau, à 25° C, on ajoute goutte à goutte 3,23 g (0,0405 mole) de $NaHCO_3$, puis goutte à goutte, 3,23 g (0,0298 mole) de chloroformiate d'éthyle. Après 1 h d'agitation, le milieu réactionnel est filtré et extrait par l'acétate d'éthyle. La phase organique est séchée sur $Na_2SO_4$ et concentrée. Le produit est obtenu avec un rendement de 57 % après chromatographie (Silice, Heptane : 80, Acétate d'éthyle : 20) ; F = 65° C. IR (KBr) $\nu$ CO : 1700 $cm^{-1}$ ; RMN$^1$H ($CDCl_3$)$\delta$ppm : 1,1-1,4 (6H) ; 1,6-1,7 (4H) ; 2,4-2,7 (2H) ; 3,9-4.4 (6H) ; 6,8-7,4 (5H).

Stade 3
[[(méthyl-2 dioxolane-1,3 yl-2)-3 propyl]-4 phényl]-3 méthoxyméthyl-5 (R) oxazolidinone-2
(numéro de code 230084)

On mélange 1 g ($3,4.10^{-3}$ mole) du composé 360274 et 0,6 g ($4,3.10^{-3}$ mole) du composé 360287 (obtenus aux stades 1 et 2) avec 50 mg (0,34 $10^{-3}$ mole) de $K_2CO_3$. Le mélange est chauffé jusqu'à ce que le $CO_2$ se dégage (150° C). Après 30 mn, le milieu réactionnel est refroidi et le produit est obtenu après chromatographie (Silice, Heptane : 70, Acétate d'éthyle : 30) avec un rendement de 100 %. Le

produit a les mêmes caractéristiques que celles obtenues par la méthode 1.

De la même manière, on obtient le [[(trifluorométhyl-2 dioxolane-1,3 yl-2)-3 propyl]-4 phenyl]-3 méthoxyméthyl-5 (R) oxazolidinone-2

Méthode 4

Stade 1
Benzoate de bromo-1 méthoxy-3 propanol-2 (S)
(numéro de code 360065)

A une solution de 13,6 g (0,07 mole) de phényl-2 méthoxyméthyl-4 dioxolane-1,3 (S) (Austr. J. Chem. 761, 29, 1976) dans 40 ml de dichloroéthane, on ajoute petit à petit 12,5 g (0,07 mole) de N-bromosuccinimide en maintenant la température à 25° C. Après 1 h d'agitation, le milieu réactionnel est versé sur l'eau. La phase organique est lavée par une solution de thiosulfate de sodium, puis à l'eau, séchée sur sulfate de magnésium et concentrée Le produit est obtenu avec 93 % de rendement, après chromatographie (Silice, Heptane : 60. Acétate d'éthyle : 40). $Eb_{0,5}$ = 95° C ; IR (microcellule) $\nu_{CO}$ : 1720 $cm^{-1}$; RMN$^1$H ($CDCl_3$)δppm : 3,4 (3H) ; 3,6-3,8 (4H) ; 5,3 (1H) ; 7,2-7,6 (3H) ; 7,9-8,1 (2H).

Stade 2
[[(méthyl-2 dioxolane-1,3 yl-2)-3 propyl]-4 phényl]-3 méthoxyméthyl-5 (R) oxazolidinone-2
(numéro de code 230084)

A une suspension de 1,96 g (0,041 mole) de NaH à 50 % dans l'huile dans 100 ml de THF, on ajoute 5 g (0,017 mole) du composé 360274 (méthode 3-stade 2), en solution dans 25 ml de THF. Après 30 mn d'agitation à 30°-40° C, on ajoute 4,64 g (0,017 mole) du composé 360065 en solution dans 25 ml de THF et le milieu réactionnel est chauffé à 50°-60° C pendant 22 h. Le milieu réactionnel est refroidi et verse sur 500 ml d'eau. La phase aqueuse est saturée par NaCl et extraite par l'acétate d'éthyle puis au chlorure de méthylène. Les phases organiques sont séchées sur $Na_2SO_4$ et concentrées. Le résidu est repris dans l'éther éthylique. Après traitement au noir animal, la solution éthérée est concentrée et le produit est recristallisé dans l'alcool isopropanol avec un rendement de 30 %. Le produit obtenu a les mêmes caractéristiques physiques que celles obtenues par la méthode 1.

Il est à noter que le composé 230084 peut également être obtenu par hydrogénation sous pression d'hydrogène en présence de Pd/C à 10 % dans l'éthanol du composé 360392 dont la synthèse est décrite à l'exemple 4 ci-après.

Exemple 4 :
[(méthyl-2 dioxolane-1,3 yl-2 propènylène-1)-4 phényl]-3 méthoxyméthyl-5 (R) oxazolidinone-2
(numéro de code 360 392)

Stade 1
(terbutyl diméthyl silyloxy méthyl)-4 nitro-1 benzène
(numéro de code 230245)

A une solution de 465 g (3,0339 moles) de paranitrobenzylalcool dans 2,5 l de DMF, on ajoute 310 g (4,559 moles) d'imidazole, puis 504 g (3,347 moles) de terbutyl diméthylchlorosilane. Après 1 h d'agitation à température ambiante le milieu réactionnel est versé sur l'eau. La phase aqueuse est extraite au chlorure de méthylène. La phase organique est séchée sur $Na_2SO_4$ et concentrée : huile ; RMN$^1$H ($CDCl_3$)δppm : 0,2 (6H) ; 1 (9H) ; 4,9 (2H) ; 7,6 (2H) ; 8,2 (2H) ; IR (microcellule) $\nu cm^{-1}$ : 1520, 1340, 1030, 840.

Stade 2
(terbutyl diméthyl silyloxy méthyl)-4 aniline
(numéro de code 230246)

A 772 ml de chlorure d'ammonium 0,1N, on ajoute 77,2 g (0,288 mole) du composé précédent (230245) et 120,8 g de poudre de fer et on chauffe à reflux 2 h. Après refroidissement on ajoute 20 ml d'ammoniaque concentrée, le milieu réactionnel est filtré et extrait au toluène. La phase organique est lavée àl'eau, séchée sur $Na_2SO_4$ et concentrée. $Eb_{0,01}$ : 88°-93° C ; RMN$^1$H ($CDCl_3$)δppm : 0,2 (6H) ; 1,05 (9H) ; 3,6 (2H) ; 4,8 (2H) ; 6,75 (2H) ; 7,2 (2H) ; IR (microcellule) $\nu$ $cm^{-1}$ : 3450,3350.

Stade 3
[(terbutyl diméthyl silyloxy méthyl)-4 phényl]-3 méthoxyméthyl-5 (R) oxazolidinone-2
(numéro de code 230247)

A une solution de 43,8 g (0,168 mole) de 370488 (exemple 3-méthode2-stade 3) dans 200 ml de toluène, on ajoute 130 ml d'une solution toluénique de phosgène 1,93 molaire, puis goutte à goutte, 37,8 g (0,252 mole) de diéthylaniline. Après refroidissement, on ajoute de l'eau glacée et la phase organique est décantée et séchée sur $Na_2SO_4$. Cette solution est alors additionnée à une solution de 40 g (0,168 mole) de 230246 et de 20,5 g (0,168 mole) de diméthylamino-4 pyridine dans 600 ml de toluène. Après $\frac{1}{2}$ h d'agitation, le milieu réactionnel est versé sur l'eau et la phase organique est lavée avec une solution de bicarbonate de sodium puis par une solution saturée en NaCl. Après concentration, le produit obtenu (84,5 g) est mis en solution dans 800 ml d'éthanol auquel est ajouté 12,2 g (0,218 mole) de KOH en pastille. Après $\frac{1}{2}$ h d'agitation, le milieu réactionnel est versé dans l'eau et extrait au $CH_2Cl_2$. La phase organique est séchée sur $Na_2SO_4$ et concentrée. Le produit attendu est obtenu après chromatographie (silice, éluant : acétate d'éthyle 30, heptane 70) avec un rendement de 63 %. $[\alpha]_D^{20}$ = - 46,2° (C = 1, $CH_3$,OH) ; IR (KBr) $\nu cm^{-1}$ : 1755, 1735 ; RMN$^1$H ($CDCl_3$)$\delta$ppm : 0 (6H) ; 1 (9H) ; 3,4 (3H) ; 3,6 (2H) ; 3,8-4,2 (2H) ; 4,7 (3H) ; 7,5 (4H) ; F <50° C.

Stade 4
[(hydroxyméthyl)-4 phényl]-3 méthoxyméthyl-5 (R) oxazolidinone-2
(numéro de code 230248)

Une solution de 29,2 g (0,083 mole) de 230247 et 7,8 g (0,025 mole) de fluorure de terbutylammonium trihydrate dans 200 ml de THF est agitée 12 h à température ordinaire et le milieu réactionnel est concentré. Le produit est obtenu après chromatographie (silice, éluant : acétate d'éthyle 50, heptane 50) : F = 65° C ; IR (KBr) $\nu cm^{-1}$ : 3400, 1750, 1720, RMN$^1$H ($CDCl_3$)$\delta$ppm : 2,4 (1H éch.) ; 3,35 (3H) ; 3,6 (2H) ; 3,8-4,2 (2H) ; 4,6 (2H) ; 7,35 (4H).

Stade 5
(carboxaldéhyde-4 phényl)-3 méthoxyméthyl-5 (R) oxazolidinone-2
(numéro de code 230256)

A une solution refroidie à - 60° C de 12,46 g (0,0982 mole) de chlorure d'oxalyle dans 80 ml de $CH_2Cl_2$ on introduit en 20 mn une solution de 12,76 g (0,1630 mole) de DMSO dans 80 ml de $CH_2Cl_2$. Après 40 mn d'agitation, on ajoute une solution de 19,6 g (0,0818 mole) de 230248 dans 80 ml de $CH_2Cl_2$ puis 41,4 g (0,409 mole) de triéthylamine. Après retour à la température ambiante, on ajoute 300 ml d'eau. La phase organique est lavée à l'eau, séchée et concentrée. Le produit a été obtenu après purification par chromatographie (silice, èluant : acétate d'éthyle 70, heptane 30) avec un rendement de 80 % ; F = 96° C ; $[\alpha]_D^{20}$ = - 73,4° (C = 1, $CH_2Cl_2$) ; IR (KBr) $\nu cm^{-1}$ : 1740, 1690 ; RMN$^1$H ($CDCl_3$) $\delta$ppm : 3,4 (3H) ; 3,7 (2H) ; 3,8-4,3 (2H) ; 4,8 (1H) ; 7,8 (4H) ; 9,8 (1H).

Stade 6
[(méthyl-2 dioxolane-1,3 yl-2 propènylène-1)-4 phényl]-3 méthoxyméthyl-5 (R) oxazolidinone-2
(numéro de code 360392)

On chauffe un mélange de 0,470 g ($2.10^{-3}$ mole) de 230256, 0,414 g ($3.10^{-3}$ mole) de $K_2CO_3$, 1,14 g (2,5. $10^{-3}$ mole) du composé phosphonium mis en oeuvre au stade 1 de l'exemple 1 en solution dans 2 ml de dioxane et 0,072 ml d'eau à 80° C pendant 3 h. Le milieu réactionnel est versé sur l'eau et extrait à l'acétate d'éthyle. La phase organique est séchée sur $MgSO_4$ et concentrée. Le produit est obtenu après chromatographie (silice, heptane : 40, acétate d'éthyle : 60), avec un rendement de 31 %.
RMN$^1$H ($CDCl_3$)$\delta$ppm : 1,3 (3H) ; 2,6 (2H) ; 3,4 (3H) ; 3,6 (2H) ; 4 (6H) ; 4,7 (1H) ; 5,7 (1H) ; 6,4 (1H) ; 7,7-7,6 (4H).

Exemple 5 :
[[(méthyl-2 dioxolane-1,3 yl-2)-3 propyl]-4 phényl]-3 méthoxyméthyl-5 oxazolidinone-2
(numéro de code 230331)

Stade 1
[[(dihydroxy-2,3 propyl) amino-4 phényl]-3 propyl]-2 méthyl-2 dioxolane-1,3
(numéro de code 230329)

A une solution de 10 g (0,045mole) de 230113 (exemple 1-stade 3) dans 60 ml d'éthanol, on ajoute 3,34 g (0,045 mole) de glycidol et laisse en agitation une nuit. Après concentration le produit est obtenu après chromatographie (silice, éluant : $CH_2Cl_2$ : 97, $CH_3OH$ : 3) ; Rendement = 44 %.

Stade 2
[[(méthyl-2 dioxolane-1,3 yl-2)-3 propyl]-4 phényl]-3 hydroxyméthyl-5 oxazolidinone-2
(numéro de code 230330)

A une solution de 5,7 g (0,019 mole) de 230329 dans 50 ml de toluène on ajoute 2,77 g (0,0023 mole) de carbonate de diéthyle à 97° C et on ajoute petit à petit 1 ml d'éthylate de sodium. Puis on chauffe à 110° C en distillant l'alcool. Au bout d' 1 h 30, le milieu réactionnel est concentré et repris dans l'eau et extrait au chloroforme. La phase organique est séchée sur $Na_2SO_4$ et concentrée. Rendement = 50 % ; F = 72° C ; IR (KBr) $\nu cm^{-1}$ : 3500, 1765.
RMN$^1$H (DMSOd$_6$)$\delta$ppm : 1,2 (3H) ; 1,6 (4H) ; 2,5 (2H) ; 3,6 (2H) ; 3,8 (4H) ; 4 (2H) ; 4,6 (1H) ; 5,2 (1H) ; 7,2 (2H) ; 7,4 (2H).

Stade 3
[[(méthyl-2 dioxolane-1,3 yl-2)-3 propyl]-4 phényl]-3 méthoxyméthyl-5 oxazolidinone-2
(numéro de code 230331)

Ce composé peut être obtenu par mise en oeuvre d'un mode opératoire identique a celui de l'exemple 2.
F = 60° C : IR (KBr) $\nu cm^{-1}$ : 1735
RMN$^1$H ($CDCl_3$)$\delta$ppm : 1,3 (3H) ; 1,6 (4H) ; 2,6 (2H) ; 3,4 (3H) ; 3,6 (2H) ; 3,9 (6H) ; 4,7 (1H) ; 7,2 (2H) ; 7,4 (2H).

Les dérivés de formule (I), ainsi que leurs sels physiologiquement acceptables éventuels ont été étudiés chez l'animal de laboratoire et ont montré des activités pharmacologiques, en particulier dans le domaine psychotrope notamment anxiolitiques et antidépresseurs potentiels.

L'activité antidépressive a été mise en évidence par le test de potentialisation du 5-HTP chez le rat selon le protocole décrit par : M. Jalfre, B. Bucher, A. Coston, G. Mocquet et R.D. Porsolt : Arch. Int. Pharmacodyn. (1982), 259, 194-221 : on détermine chez le rat la dose de produit qui, administrée par voie orale, provoque chez 50 % des animaux ($DE_{50}$) l'apparition de tremblements généralisés ou des stéréotypies (pianotage, mouvements de tête) consécutifs à l'administration par voie intrapéritonéale 1 h après le premier traitement d'une dose de 120 mg d'hydroxy-5 tryptophane (5-HTP).

Le résultat obtenu avec un composé selon l'invention dans le test précédent est donné, à titre d'exemple, dans le tableau ci-après .

TABLEAU

| Composé testé Numéro de code | $DE_{50}$mg/kg |
|---|---|
| 230084 | 0,97 |
| TOLOXATONE | 30 |

Le résultat qui précède montre que les composés objet de la présente demande peuvent être utilisés pour la préparation de médicaments psychotropes et notamment d'antidépresseurs potentiels, ces médicaments trouvant leur emploi en thérapeutique, notamment pour le traitement des états dépressifs endogène et exogène.

Ces médicaments peuvent être administrés à l'homme ou à tout animal à sang chaud, sous diverses formes pharmaceutiques bien connues dans la technique et notamment sous la forme de compositions formulées en vue de leur administration par voie orale, injectable ou rectale. Pour l'administration par voie orale, lesdites compositions peuvent prendre la forme de comprimés, dragées ou gélules préparées par les techniques habituelles utilisant des supports et excipients connus tels que des agents liants, des charges, des lubrifiants et des agents de désintégration ; elles peuvent également prendre la forme de solutions, sirop ou suspensions.

Pour l'administration sous forme de soluté injectable, les compositions selon l'invention peuvent prendre la forme de solutions, suspensions ou émulsions injectables comprenant un véhicule liquide huileux ou aqueux acceptable.

Pour l'administration par voie rectale, les compositions peuvent prendre la forme de suppositoire comprenant les bases habituelles pour suppositoires.

La dose thérapeutique active des principes actifs dépend notamment de la voie d'administration, du poids corporel du patient et de la puissance thérapeutique des principes actifs mis en oeuvre.

Généralement, par voie orale, les doses administrées pourront atteindre 10 mg/kg/jour de principe actif (en une ou plusieurs prises) ; par voie injectable, elles pourront atteindre 1 mg/kg/jour (en une ou plusieurs prises) ; par voie rectale, elles pourront atteindre 5 mg/kg/jour de principe actif (en un ou plusieurs suppositoires).

## Revendications

**Revendications pour les Etats contractants suivants : AT, BE, CH, DE, DK, FR, GB, IT, LI, LU, NL, SE**

**1.** Dérivés répondant à la formule :

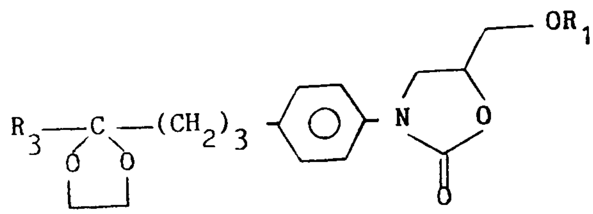

(I)

où :

- $R_1$ représente H ou alkyle en $C_1$-$C_4$ ; et
- $R_3$ représente un groupe alkyle en $C_1$-$C_4$ ou $CF_3$,

ces dérivés se présentant sous la forme d'énantiomères ou de mélanges d'énantiomères, y compris de mélanges racémiques,

ainsi que les sels d'addition d'acide éventuels de ces dérivés.

**2.** Dérivés et sels selon la revendication 1, pour lesquels $R_1$ = H ou $CH_3$ et $R_3$ = alkyle en $C_1$-$C_4$.

**3.** Dérivés selon la revendication 1, pour lesquels $R_1$ = $CH_3$ et $R_3$ = $CH_3$ ou $CF_3$.

**4.** Dérivé selon la revendication 1, pour lequel $R_1$ = $CH_3$ et $R_3$ = $CH_3$.

**5.** Dérivé selon la revendication 4, pour lequel l'atome de carbone asymétrique est de configuration (R).

**6.** Composition pharmaceutique, caractérisée en ce qu'elle comprend un excipient physiologiquement acceptable et au moins un composé choisi parmi ceux objet de l'une quelconque des revendications 1 à 5.

**7.** Utilisation des composés objet des revendications 1 à 5 pour la préparation de médicaments.

**8.** Procédé de préparation des dérivés de formule (I) et sels selon la revendication 1, caractérisé en ce qu'il comprend :

a) la condensation du composé de formule :

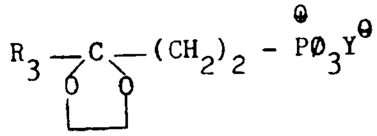

où Y = halogène et $R_3$ a la même signification que dans la formule (I), sur les composés de formule :

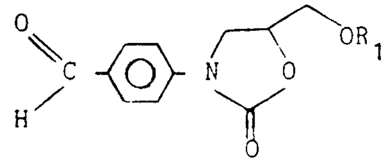

où $R_1$ = alkyle en $C_1$-$C_4$, suivie de l'hydrogénation de la double liaison des composés ainsi obtenus de formule :

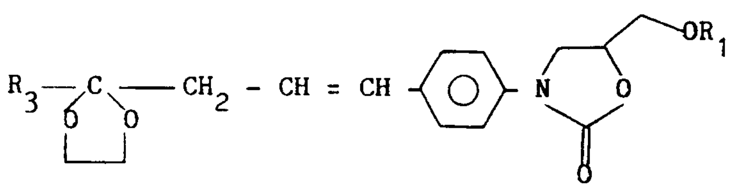

où $R_1$ et $R_3$ ont les mêmes significations que ci-dessus ;
b) la condensation d'un carbonate d'alkyle en $C_1$-$C_4$ sur les composés de formule :

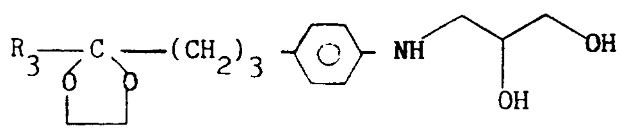

où $R_3$ a la même signification que dans la formule (I) ;
c) l'alcoylation par un halogénure d'alkyle en $C_1$-$C_4$ des composés de formule :

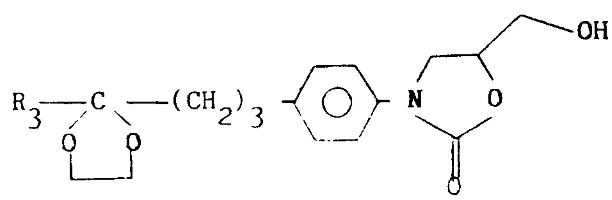

où $R_3$ a la même signification que dans la formule (I) ;

d) l'acétalisation des composés de formule :

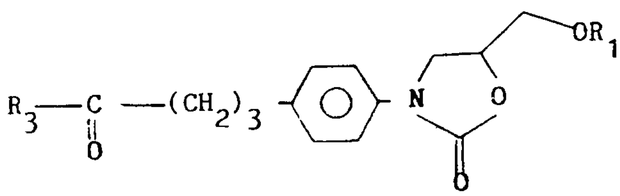

où $R_1$ = alkyle en $C_1$-$C_4$ et $R_3$ = alkyle en $C_1$-$C_4$, par HO-$(CH_2)_2$-OH ;

e) la condensation sur les composés de formule :

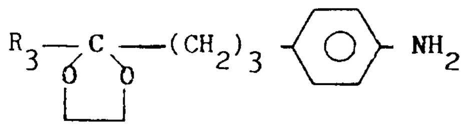

où $R_3$ a la même signification que dans la formule (I), d'un composé de formule :

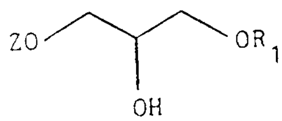

où Z = Ts ou Ms et $R_1$ = alkyle en $C_1$-$C_4$, en présence de phosgène ;

f) la condensation sur les composés de formule :

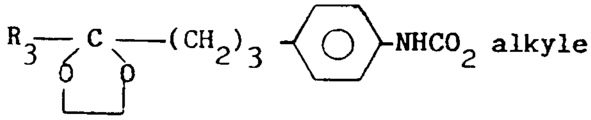

où $R_3$ a la même signification que dans la formule (I), d'un composé de formule :

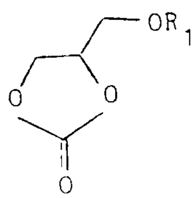

ou

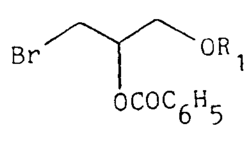

où $R_1$ = alkyle en $C_1$-$C_4$ ; ou

g) la condensation sur les composés de formule :

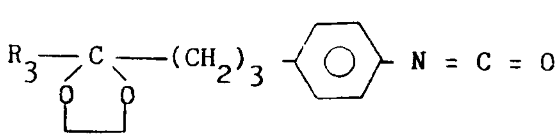

où $R_3$ a la même signification que dans la formule (I), d'un composé de formule :

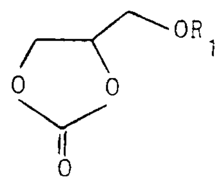

où $R_1$ = alkyle en $C_1$-$C_4$ ;
les réactions a) à g) ci-dessus étant éventuellement suivies de la salification par un acide des composés obtenus.

**Revendications pour les Etats contractants suivants : ES, GR**

**1.** Procédé de préparation des dérivés répondant à la formule :

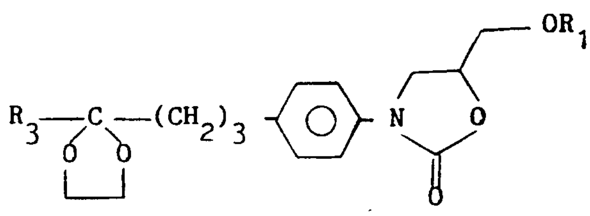

(I)

où :

- $R_1$ représente H ou alkyle en $C_1$-$C_4$ ; et
- $R_3$ représente un groupe alkyle en $C_1$-$C_4$ ou $CF_3$,

ces dérivés se présentant sous la forme d'énantiomères ou de mélanges d'énantiomères, y compris de mélanges racémiques,
ainsi que des sels d'addition d'acide éventuels de ces dérivés, caractérisé en ce qu'il comprend :

a) la condensation du composé de formule :

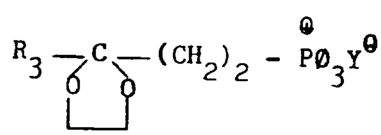

où Y = halogène et $R_3$ a la même signification que dans la formule (I), sur les composés de formule :

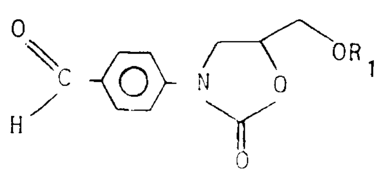

où $R_1$ = alkyle en $C_1$-$C_4$, suivie de l'hydrogénation de la double liaison des composés ainsi obtenus de formule :

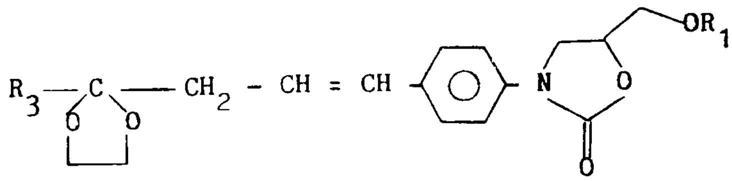

où $R_1$ et $R_3$ ont les mêmes significations que ci-dessus ;
b) la condensation d'un carbonate d'alkyle en $C_1$-$C_4$ sur les composés de formule :

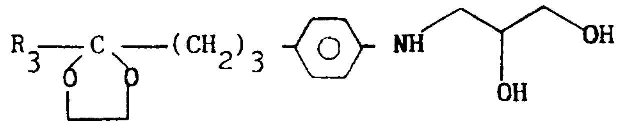

où $R_3$ a la même signification que dans la formule (I) ;
c) l'alcoylation par un halogénure d'alkyle en $C_1$-$C_4$ des composés de formule :

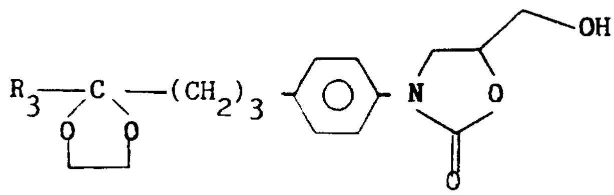

où $R_3$ a la même signification que dans la formule (I) ;
d) l'acétalisation des composés de formule :

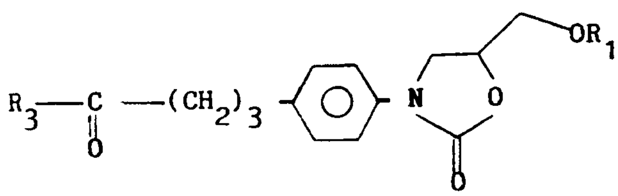

où $R_1$ = alkyle en $C_1$-$C_4$ et $R_3$ = alkyle en $C_1$-$C_4$, par $HO-(CH_2)_2-OH$ ;
e) la condensation sur les composés de formule :

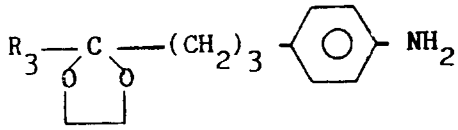

où $R_3$ a la même signification que dans la formule (I), d'un composé de formule :

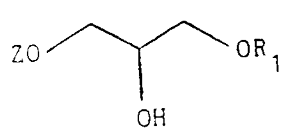

où Z = Ts ou Ms et $R_1$ = alkyle en $C_1$-$C_4$, en présence de phosgène ;
f) la condensation sur les composés de formule :

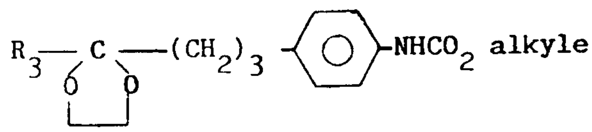

où $R_3$ a la même signification que dans la formule (I), d'un composé de formule :

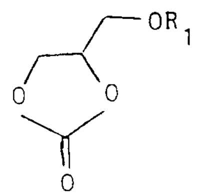

ou

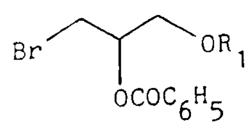

où $R_1$ = alkyle en $C_1$-$C_4$ ; ou
g) la condensation sur les composés de formule :

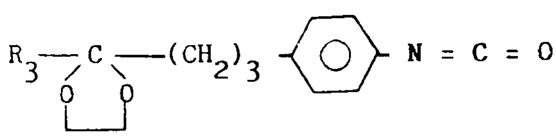

où $R_3$ a la même signification que dans la formule (I), d'un composé de formule :

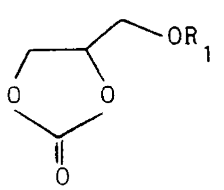

où $R_1$ = alkyle en $C_1$-$C_4$ ;
les réactions a) à g) ci-dessus étant éventuellement suivies de la salification par un acide des composés obtenus.

**2.** Procédé selon la revendication 1, pour la préparation de dérivés et sels de formule (I) pour lesquels $R_1$ = H ou $CH_3$ et $R_3$ = alkyle en $C_1$-$C_4$.

**3.** Procédé selon la revendication 1, pour la préparation de dérivés de formule (I) pour lesquels $R_1$ = $CH_3$ et $R_3$ = $CH_3$ ou $CF_3$.

**4.** Procédé selon la revendication 1, pour la préparation du dérivé de formule (I) pour lequel $R_1$ = $CH_3$ et $R_3$ = $CH_3$.

**5.** Procédé selon la revendication 4, pour la préparation du dérivé de formule (I) pour lequel l'atome de carbone asymétrique est de configuration (R).

## Claims

### Claims for the following Contracting States : AT, BE, CH, DE, DK, FR, GB, IT, LI, LU, NL, SE,

1. The derivatives of the formula :

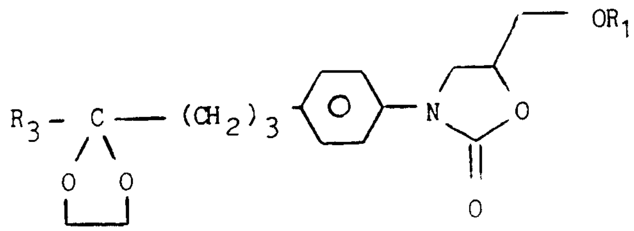

(I)

wherein :
- $R_1$ is H or $C_1$-$C_4$ alkyl ; and
- $R_3$ is a $C_1$-$C_4$ alkyl or $CF_3$ group,

these derivatives being under the form of enantiomers or mixtures of enantiomers, including the racemic mixtures, as well as the optional acid addition salts of said derivatives.

2. The derivatives and salts according to Claim 1, wherein $R_1$ = H or $CH_3$ and $R_3$ = $C_1$-$C_4$ alkyl.

3. The derivatives according to Claim 1, wherein $R_1$ = $CH_3$ and $R_3$ = $CH_3$ or $CF_3$.

4. The derivative according to Claim 1, wherein $R_1$ = $CH_3$ and $R_3$ = $CH_3$.

5. The derivative according to Claim 4, wherein the asymetric carbon atom has the (R) configuration.

6. A pharmaceutical composition, characterized in that it contains a physiologically acceptable excipient and at least one compound selected among those according to any one of Claims 1 to 5.

7. The use of the compounds according to Claims 1 to 5 for the preparation of drugs.

8. A process for the preparation of derivatives of formula (I) and salts according to Claim 1, characterized in that it comprises :

a) condensing a compound of formula :

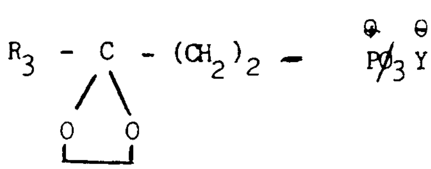

wherein Y = halogen and $R_3$ has the same meaning as in formula (I), with compounds of formula :

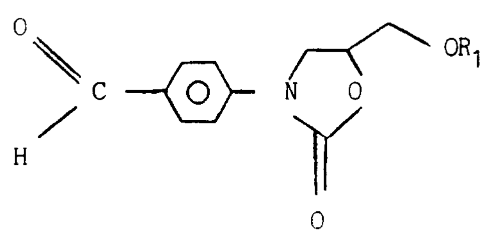

wherein $R_1$ = $C_1$-$C_4$ alkyl, followed by hydrogenating the double bond of the thus obtained compounds of formula :

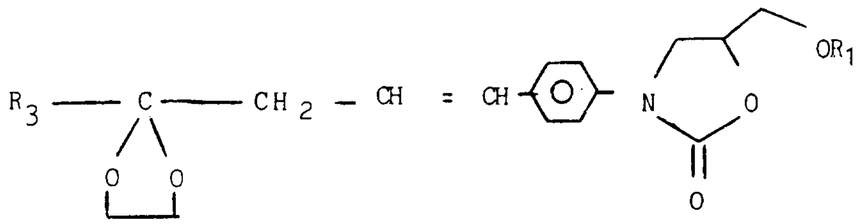

wherein $R_1$ and $R_3$ have the same meanings as above ;
b) condensing a $C_1$-$C_4$ alkyl carbonate with compounds of formula :

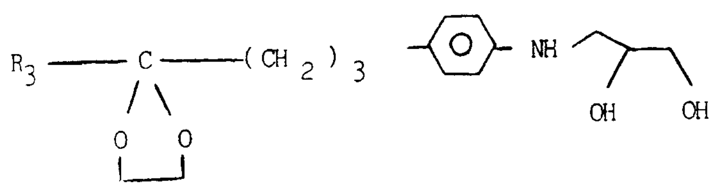

wherein $R_3$ has the same meaning as in formula (I) ;
c) alkylating with a $C_1$-$C_4$ alkyl halide compounds of formula:

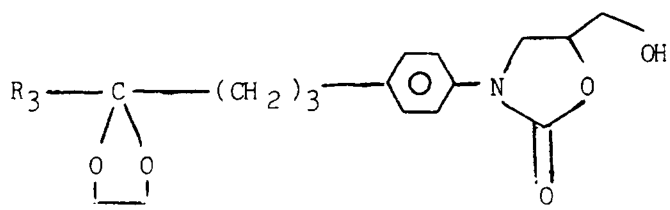

wherein $R_3$ has the same meaning as in formula (I) ;
d) acetalising compounds of formula :

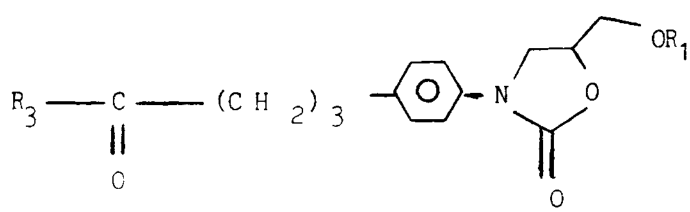

wherein $R_1$ = $C_1$-$C_4$ alkyl and $R_3$ = $C_1$-$C_4$ alkyl, with $HO-(CH_2)_2-OH$;

e) condensing with compounds of formula :

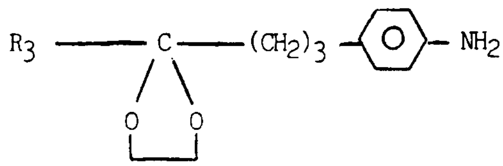

wherein $R_3$ has the same meaning as in formula (I), a compound of formula :

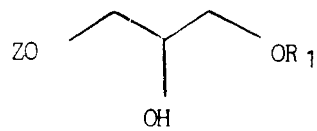

wherein Z = Ts or Ms and $R_1$ = $C_1$-$C_4$ alkyl, in the presence of phosgene ;
f) condensing with compounds of formula :

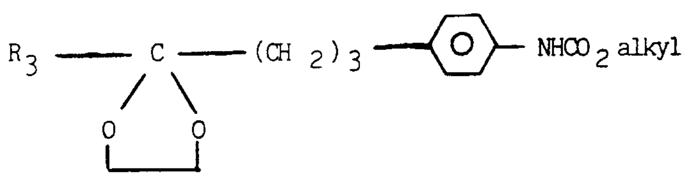

wherein $R_3$ has the same meaning as in formula (I), a compound of formula :

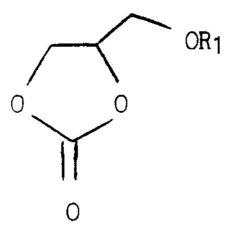

or

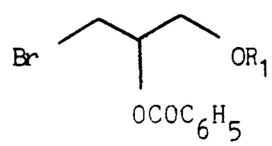

wherein $R_1$ = $C_1$-$C_4$ alkyl ; or
g) condensing with compounds of formula :

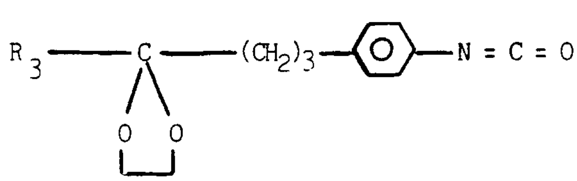

wherein $R_3$ has the same meaning as in formula (I), a compound of formula :

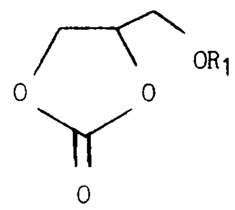

wherein $R_1$ = $C_1$-$C_4$ alkyl ;
the above mentioned reactions a) to g) being optionally followed by the step of salifying the obtained compounds with an acid.

**Claims for the following Contracting States : ES, GR**

**1.** A process for the preparation of the derivatives of the formula :

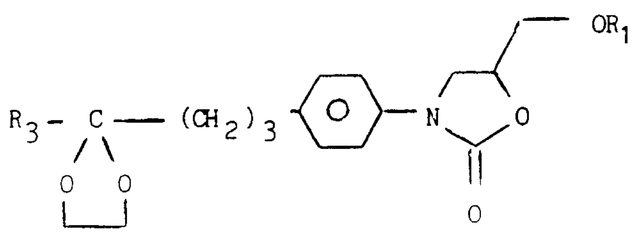

(I)

wherein :
- $R_1$ is H or $C_1$-$C_4$ alkyl ; and
- $R_3$ is a $C_1$-$C_4$ alkyl or $CF_3$ group,

these derivatives being under the form of enantiomers or mixtures of enantiomers, including the racemic mixtures, as well as of the optional acid addition salts of said derivatives,
characterized in that it comprises :
a) condensing a compound of formula :

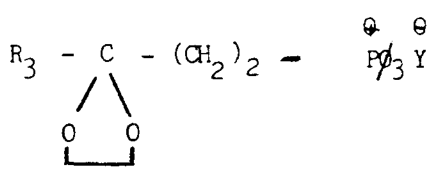

wherein Y = halogen and $R_3$ has the same meaning as in formula (I),
with compounds of formula :

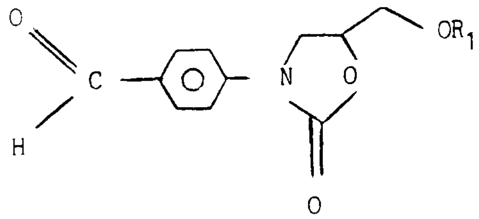

wherein $R_1$ = $C_1$-$C_4$ alkyl, followed by hydrogenating the double bond of the thus obtained compounds of formula :

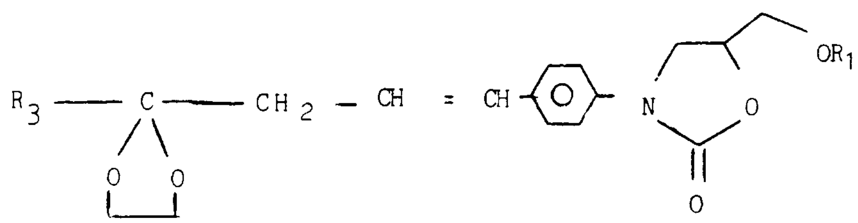

wherein $R_1$ and $R_3$ have the same meanings as above ;
b) condensing a $C_1$-$C_4$ alkyl carbonate with compounds of formula :

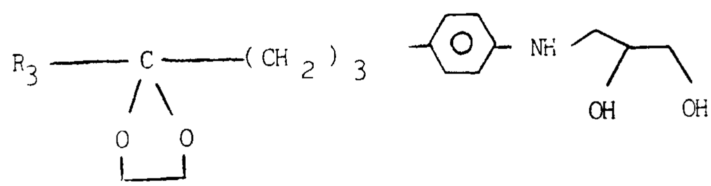

wherein $R_3$ has the same meaning as in formula (I) ;
c) alkylating with a $C_1$-$C_4$ alkyl halide compounds of formula:

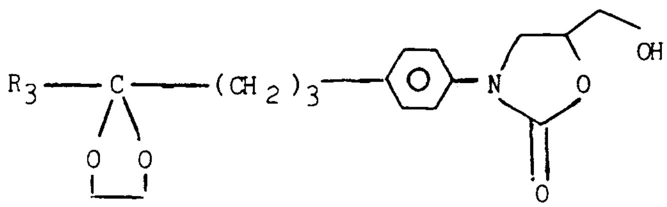

wherein $R_3$ has the same meaning as in formula (I) ;
d) acetalising compounds of formula :

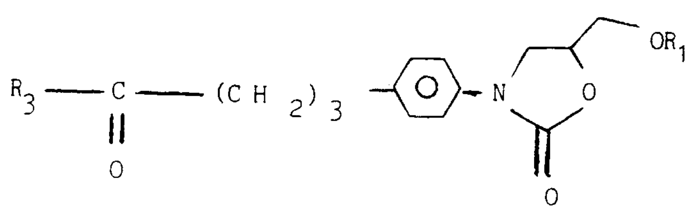

wherein $R_1$ = $C_1$-$C_4$ alkyl and R = $C_1$-$C_4$ alkyl, with $HO\text{-}(CH_2)_2\text{-}OH$ ;
e) condensing with compounds of formula :

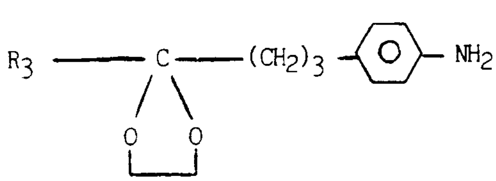

wherein $R_3$ has the same meaning as in formula (I), a compound of formula :

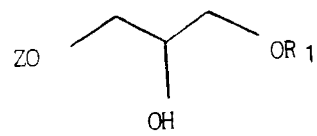

wherein Z = Ts or Ms and $R_1$ = $C_1$-$C_4$ alkyl, in the presence of phosgene ;
f) condensing with compounds of formula :

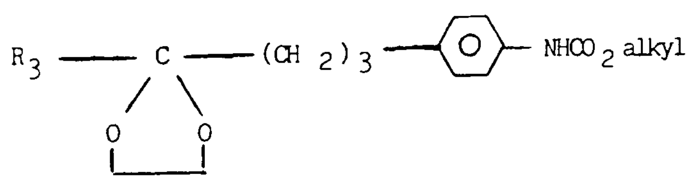

wherein $R_3$ has the same meaning as in formula (I), a compound of formula :

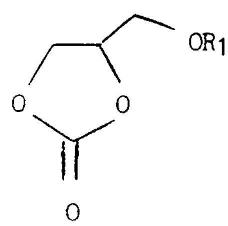

or

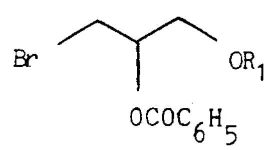

wherein $R_1$ = $C_1$-$C_4$ alkyl ; or
g) condensing with compounds of formula :

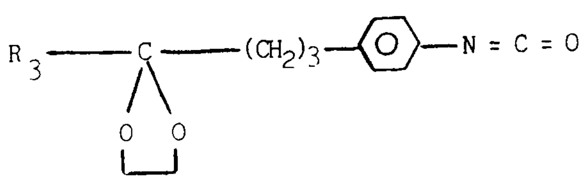

wherein $R_3$ has the same meaning as in formula (I), a compound of formula :

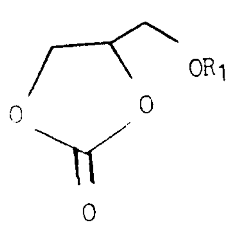

wherein $R_1$ = $C_1$-$C_4$ alkyl ;
the above mentioned reactions a) to g) being optionally followed by the step of salifying the obtained compounds with an acid.

2. The process according to Claim 1, for the preparation of derivatives and salts of formula (I) wherein $R_1$ = H or $CH_3$ and $R_3$ = $C_1$-$C_4$ alkyl.

3. The process according to Claim 1, for the preparation of derivatives of formula (I) wherein $R_1$ = $CH_3$ and $R_3$ = $CH_3$ or $CF_3$.

4. The process according to Claim 1, for the preparation of the derivative of formula (I) wherein $R_1$ = $CH_3$ and $R_3$ = $CH_3$.

5. The process according to Claim 4, for the preparation of the derivative of formula (I) wherein the asymetric carbon atom has the (R) configuration.

**Patentansprüche**
**Patentansprüche für folgende Vertragsstaaten : AT, BE, CH, DE, DK, FR, GB, IT, LI, LU, NL, SE**

1. Derivate gemäß der Formel:

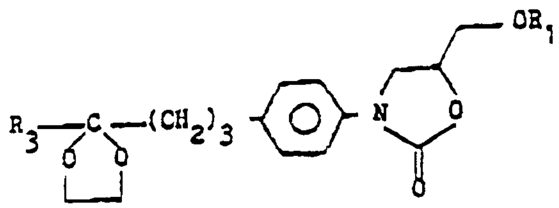

(I)

worin:
- $R_1$ H oder $C_1$-$C_4$-Alkyl bedeutet; und
- $R_3$ eine $C_1$-$C_4$-Alkylgruppe oder $CF_3$ bedeutet,

wobei diese Derivate in Form von Enantiomeren oder Enantiomeren-Gemischen einschließlich der recemischen Gemische vorliegen,
sowie gegebenenfalls die Säureadditionssalze dieser Derivate.

2. Derivate und Salze nach Anspruch 1, worin $R_1$ = H oder $CH_3$ und $R_3$ = $C_1$-$C_4$-Alkyl.

3. Derivate nach Anspruch 1, worin $R_1$ = $CH_3$ und $R_3$ = $CH_3$ oder $CF_3$.

4. Derivat nach Anspruch 1, worin $R_1$ = $CH_3$ und $R_3$ = $CH_3$.

5. Derivat nach Anspruch 4, worin das asymmetrische Kohlenstoffatom die (R)-Konfiguration hat.

6. Pharmazeutische Zusammensetzung, **dadurch gekennzeichnet**, daß sie ein physiologisch annehmbares Excipiens und mindestens eine Verbindung umfaßt, die gewählt ist aus den Verbindungen, die Gegenstand eines der Ansprünche 1 bis 5 sind.

7. Verwendung der Verbindungen, die Gegenstand der Ansprüche 1 bis 5 sind, zur Herstellung von Arzneimitteln.

8. Verfahren zur Herstollung von Derivaten der Formel (I) und Salzen nach Anspruch 1, **dadurch gekennzeichnet**, daß es umfaßt:
a) die Kondensation der Verbindung der Formel:

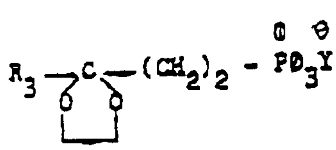

worin Y = Halogen und $R_3$ die gleiche Bedeutung wie in Formel (I) hat, mit den Verbindungen der Formel:

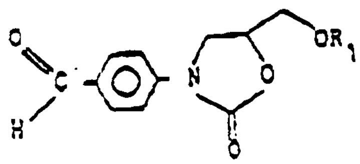

worin $R_1$ = $C_1$-$C_4$-Alkyl, gefolgt von der Hydrierung der Doppelbindung der so erhaltenen Verbindungen der Formel:

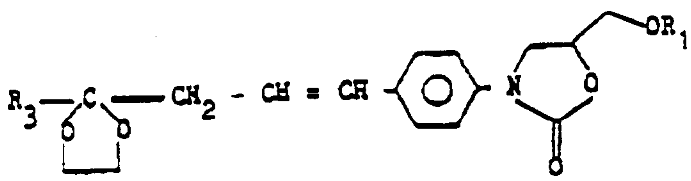

worin $R_1$ und $R_3$ die gleichen Bedeutungen wie oben haben;
b) die Kondensation eines $C_1$-$C_4$-Alkylcarbonats mit den Verbindungen der Formel:

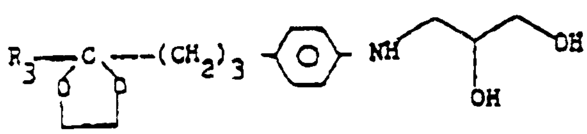

worin $R_3$ die gleiche Bedeutung wie in Formel (I) hat;
c) die Alkylierung von Verbindungen der Formel:

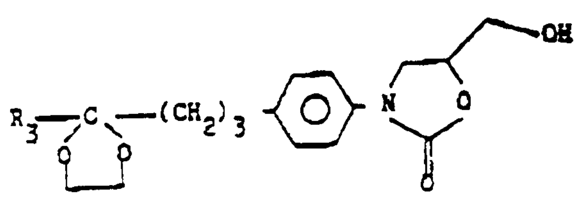

worin $R_3$ die gleiche Bedeutung wie in Formel (I) hat, mit einem $C_1$-$C_4$-Alkylhalogenid;
d) die Acetalisierung von Verbindungen der Formel:

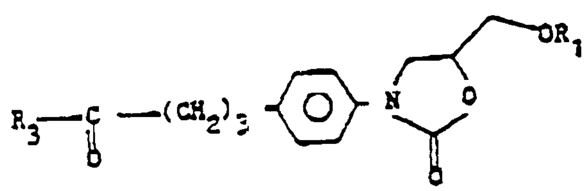

worin $R_1$ = $C_1$-$C_4$-Alkyl und $R_3$ = $C_1$-$C_4$-Alkyl, mit HO-$(CH_2)_2$-OH;

e) die Kondensation der Verbindungen der Formel:

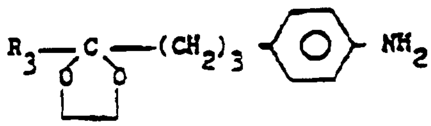

worin $R_3$ die gleiche Bedeutung wie in Formel (I) hat, mit einer Verbindung der Formel:

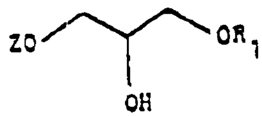

worin Z = Ts oder Ms und $R_1$ = $C_1$-$C_4$-Alkyl, in Gegenwart von Phosgen;
f) die Kondensation der Verbindungen der Formel:

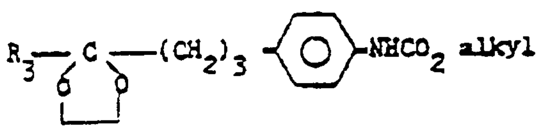

worin $R_3$ die gleiche Bedeutung wie in Formel (I) hat, mit einer Verbindung der Formel:

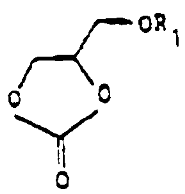

oder

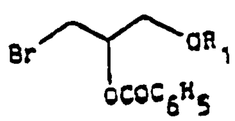

worin $R_1$ = $C_1$-$C_4$-Alkyl; oder
g) die Kondensation der Verbindungen der Formel:

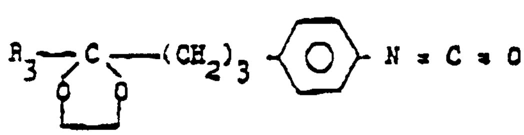

worin $R_3$ die gleiche Bedeutung wie in Formel (I) hat, mit einer Verbindung der Formel:

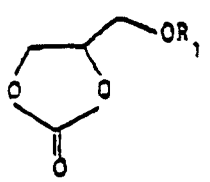

worin $R_1$ = $C_1$-$C_4$-Alkyl;
wobei auf die vorstehenden Reaktionen a) bis g) gegebenenfalls die Bildung eines Salzes der erhaltenen Verbindungen mit einer Säure erfolgt.

**Patentansprüche für folgende Vertragsstaaten : ES, GR**

1. Verfahren zur Herstellung von Derivaten gemäß der Formel:

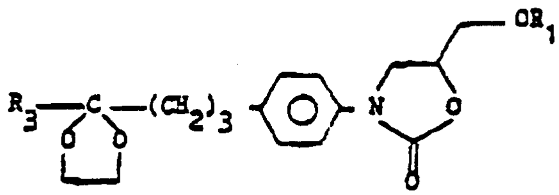

(I)

worin:

- $R_1$ H oder $C_1$-$C_4$-Alkyl bedeutet; und
- $R_3$ eine $C_1$-$C_4$-Alkylgruppe oder $CF_3$ bedeutet,

wobei diese Derivate in Form von Enantiomeren oder Enantiomeren-Gemischen einschließlich der racemischen Gemische vorliegen,
sowie gegebenenfalls von Säureadditionssalzen dieser Derivate, **dadurch gekennzeichnet**, daß es umfaßt:

a) die Kondensation der Verbindung der Formel:

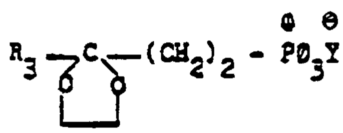

worin Y = Halogen und $R_3$ die gleiche Bedeutung wie in Formel (I) hat, mit den Verbindungen der Formel:

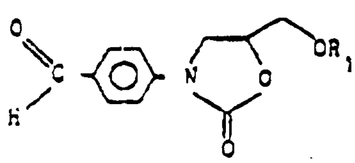

worin $R_1$ = $C_1$-$C_4$-Alkyl, gerolgt von der Hydrierung der Doppelbindung der so erhaltenen Verbindungen der Formel:

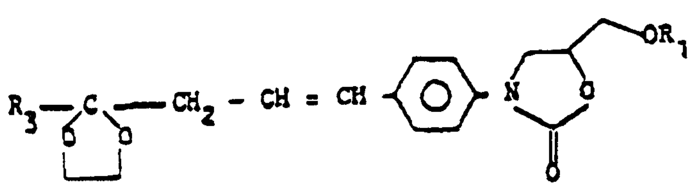

worin $R_1$ und $R_3$ die gleichen Bedeutungen wie oben haben;

b) die Kondensation eines $C_1$-$C_4$-Alkylcarbonats mit den Verbindungen der Formel:

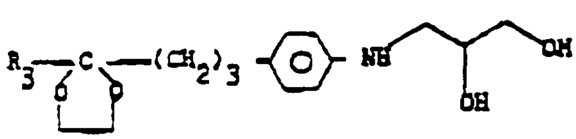

worin $R_3$ die gleiche Bedeutung wie in Formel (I) hat;
c) die Alkylierung von Verbindungen der Formel:

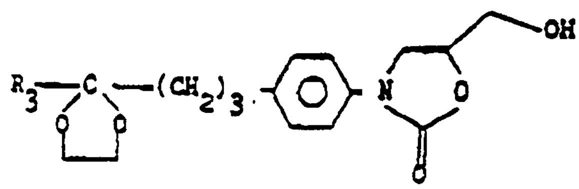

worin $R_3$ die gleiche Bedeutung wie in Formel (I) hat, mit einem $C_1$-$C_4$-Alkylhalogenid;
d) die Acetalisierung von Verbindungen der Formel:

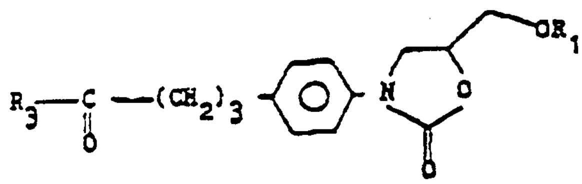

worin $R_1$ = $C_1$-$C_4$-Alkyl und $R_3$ = $C_1$-$C_4$-Alkyl, mit HO-$(CH_2)_2$-OH;
e) die Kondensation der Verbindungen der Formel:

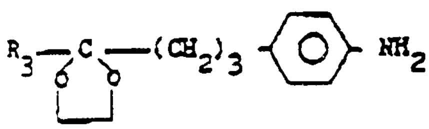

worin $R_3$ die gleiche Bedeutung wie in Formel (I) hat, mit einer Verbindung der Formel:

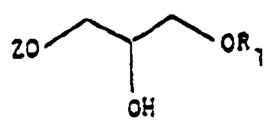

worin Z = Ts oder Ms und $R_1$ = $C_1$-$C_4$-Alkyl, in Gegenwart von Phosgen;
f) die Kondensation der Verbindungen der Formel:

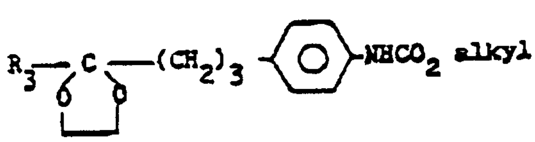

worin $R_3$ die gleiche Bedeutung wie in Formel (I) hat, mit einer Verbindung der Formel:

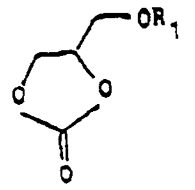

oder

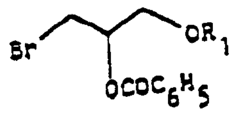

worin $R_1$ = $C_1$-$C_4$-Alkyl; oder

g) die Kondensation der Verbindungen der Formel:

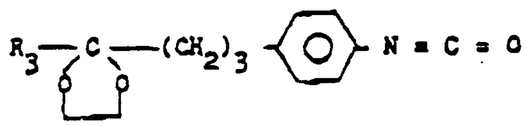

worin $R_3$ die gleiche Bedeutung wie in Formel (I) hat, mit einer Verbindung der Formel:

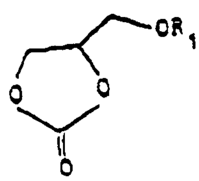

worin $R_1$ = $C_1$-$C_4$-Alkyl;

wobei auf die vorstehenden Reaktionen a) bis g) gegebenenfalls die Bildung eines Salzes der erhaltenen Verbindungen mit einer Säure erfolgt.

**2.** Verfahren nach Anspruch 1, zur Herstellung von Derivaten und Salzen der Formel (I), worin $R_1$ = H oder $CH_3$ und $R_3$ = $C_1$-$C_4$-Alkyl.

**3.** Verfahren nach Anspruch 1, zur Herstellung von Derivaten der Formel (I), worin $R_1$ = $CH_3$ und $R_3$ = $CH_3$ oder $CF_3$.

**4.** Verfahren nach Anspruch 1, zur Herstellung des Derivate der Formel (I), worin $R_1$ = $CH_3$ und $R_3$ = $CH_3$.

**5.** Verfahren nach Anspruch 4, zur Herstellung des Derivate der Formel (I), worin das asymmetrische Kohlenstoffatom die (R)-Konfiguration hat.